(19) Europäisches Patentamt
European Patent Office
Office européen des brevets

(11) Veröffentlichungsnummer: **0 037 944**
**B1**

(12) **EUROPÄISCHE PATENTSCHRIFT**

(45) Veröffentlichungstag der Patentschrift: **30.05.84**

(51) Int. Cl.³: **B 01 D 53/34**, B 01 D 53/36, B 01 J 20/02, A 61 F 5/44

(21) Anmeldenummer: **81102337.3**

(22) Anmeldetag: **27.03.81**

# (54) Verfahren und Mittel zur Adsorption und katalytischen Zerlegung übelriechender Darmgase.

(30) Priorität: **03.04.80 DE 3013255**

(43) Veröffentlichungstag der Anmeldung: **21.10.81 Patentblatt 81/42**

(45) Bekanntmachung des Hinweises auf die Patenterteilung: **30.05.84 Patentblatt 84/22**

(84) Benannte Vertragsstaaten: **AT BE CH DE FR GB IT LI LU NL SE**

(56) Entgegenhaltungen:
**DD - A - 129 160**
**DE - A - 2 209 564**
**DE - A - 2 650 407**
**DE - A - 2 752 317**
**DE - A - 3 018 279**
**DE - B - 1 144 692**
**DE - B - 1 277 817**
**GB - A - 1 235 874**
**GB - A - 1 550 960**

(73) Patentinhaber: **Fraunhofer-Gesellschaft zur Förderung der angewandten Forschung e.V., Leonrodstrasse 54, D-8000 München 19 (DE)**

(72) Erfinder: **Chmiel, Horst, Prof. Dr.-Ing., Paracelsusstrasse 14, D-7250 Leonberg (DE)**
Erfinder: **Hellwig, Günter, Dr. rer. nat., Im Langen Hau 29, D-7000 Stuttgart 80 (DE)**

(74) Vertreter: **Kraus, Walter, Dr. et al, Patentanwälte Kraus & Weisert Irmgardstrasse 15, D-8000 München 71 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 037 944 B1

**Beschreibung**

Die Erfindung betrifft ein Verfahren zur Adsorption und Zerlegung übelriechender Darmgase, ein Mittel zur Durchführung des Verfahrens und ein Verfahren zur Herstellung eines hochporösen Feststoffs.

Ein Kunstafter oder *Anus praeternaturalis* muss oft zur Behandlung bestimmter, manchmal bösartiger Darmerkrankungen angelegt werden. Bedingt durch immer häufigere Darmkrebserkrankungen nimmt die Anzahl der Personen, die einen *Anus praeternaturalis* tragen, ständig zu.

Eine Schwierigkeit, die beim Tragen eines *Anus praeter* auftritt, ist die Tatsache, dass der Stuhl und die Darmgase nicht mehr willkürlich zurückgehalten werden können. Der Stuhl wird in einer mit Gummiringen versehenen, dem Körper fest aufliegenden Pelotte oder in einem aufklebbaren Plastikbeutel aufgefangen. Nach Gewöhnung sind die Träger eines *Anus praeter* auch gesellschaftlich nicht behindert.

Eine Schwierigkeit, die mit dem Tragen eines Plastikbeutels verbunden ist, ist die Entfernung der auftretenden Darmgase. Die Darmgase sind der gasförmige Darminhalt und bestehen teils aus verschluckter Luft. Teilweise sind sie durch Umsetzungen im Darm entstanden. Die von den Verdauungsvorgängen in den oberen Darmabschnitten unberührt gelassenen Kohlenhydrate und Eiweisskörper werden vorwiegend im Dickdarm durch Bakterien zu gasförmigen Produkten abgebaut, die zwar einen hohen Energiegehalt aufweisen, aber für den Organismus nicht mehr nutzbar gemacht werden können. Solche Produkte sind Wasserstoff, Schwefelwasserstoffe, Mercaptane, Kohlenwasserstoffe (Methan), Kohlendioxid. Die Zusammensetzung der Darmgase ist abhängig von der Beschaffenheit der Nahrung, aber fast immer sind in den Darmgasen sehr übelriechende Stoffe vorhanden.

So entstehen übelriechende Thiole, besonders Methanthiol, während des Verdauungsvorgangs bei der bakteriellen Eiweisszersetzung im menschlichen Magen/Darm-Bereich. Die Geruchsschwelle für die menschliche Nase liegt für Methanthiol ($CH_3SH$) bei 0,002 ppb (part per billion), d.h. 2 Teile $CH_3SH$ können pro $10^{12}$ Teile Neutralgas schon unangenehm wahrgenommen werden. Bei Kolostomiepatienten treten methanthiolhaltige Darmgase unkontrolliert aus, so dass grosse Geruchsbelästigungen entstehen können. Das Darmgas ist ein Gemisch aus hauptsächlich Methan, $CO_2$, schwefelhaltigen Kohlenwasserstoffverbindungen (z.B. $CS_2$, $CH_3COSH$), wie Thiole, in fast 100% gesättigter Wasserdampfatmosphäre.

Das Auftreten der übelriechenden Gase ist für den Träger des *Anus praeter* eine grosse Belastung, da er dauernd das Gefühl haben muss, dass er riecht. Man hat in der Vergangenheit versucht, die Gerüche zu entfernen, indem man an die Öffnungen der Beutel Kohlefilter anbrachte. Diese Filter besitzen jedoch den Nachteil, dass die Kohle sehr schnell belegt ist. Die Kohle adsorbiert nicht nur die übelriechenden Stoffe, die überwiegend Schwefelprodukte, insbesondere Mercaptane, enthalten, sondern gleichzeitig auch Wasserdampf, Kohlenstoffmonoxid und Kohlenstoffdioxid. Diese sind in den Darmgasen in grösseren Mengen enthalten. Dadurch wird die Kohle sehr schnell belegt und verliert auch ihre Adsorptionsfähigkeit für die übelriechenden Stoffe. Ausserdem wird die Adsorbensoberfläche durch die auftretenden Schwefelverbindungen schnell vergiftet. Dies bedeutet, dass die Filter sehr häufig ausgetauscht werden müssen oder dass ihre Adsorptionsfähigkeit im Verlauf der Zeit sehr stark nachlässt und sie nur kurze Zeit, je nach Adsorbensmenge, einige Minuten bis 1 bis 2 Stunden wirken.

Aus der GB-A Nr. 1235874 ist es bekannt, Aktivkohle mit 3 bis 15 Gew.-% Eisen oder Zink zu belegen.

Mit Metallsalzlösungen imprägnierte Aktivkohlen sind schon seit langem im Handel und teilweise auch patentrechtlich geschützt. Die imprägnierten Aktivkohlen zeigen jedoch noch immer zwei Nachteile: Zum einen sind sie nicht beliebig lagerfähig, und zum anderen ist ihre Haltezeit (auch Adsorptionskapazität) noch zu gering, d.h. sie ist noch wesentlich zu verbessern (bis zu einem Faktor von $1 \times 10^2$).

Es besteht daher ein grosser Bedarf an Stoffen, die selektiv aus den Darmgasen nur die übelriechenden Stoffe adsorbieren und nichtriechende Stoffe, wie Methan, Wasserstoff und Wasserdampf, Kohlenstoffmonoxid und Kohlenstoffdioxid, nicht adsorbieren.

Der vorliegenden Erfindung liegt die Aufgabe zugrunde, ein Verfahren sowie ein Mittel zur Adsorption und katalytischen Zerlegung übelriechender Darmgase in- und ausserhalb des Darms und ein Verfahren zur Herstellung des Mittels zur Verfügung zu stellen. Das erstgenannte Verfahren und das Mittel sollen möglichst während langer Zeit wirken und eine Adsorption der übelriechenden Darmgase während mindestens 12 Stunden gewährleisten.

Weiterhin soll die Menge and Adsorbens pro Patient klein gehalten werden und möglichst gering sein. Ausserdem sollen alle im Darmgas vorkommenden, übelriechenden und giftigen Substanzen entfernt bzw. unschädlich bemacht werden.

Gegenstand der Erfindung ist somit ein Verfahren zur Adsorption und katalytischen Zerlegung übelriechender, Wasserdampf, Methanol, Kohlenstoffmonoxid und Kohlenstoffdioxid und verschiedene schwefelhaltige Gase, insbesondere Mercaptane, enthaltender Darmgase, das dadurch gekennzeichnet ist, dass man die Darmgase durch einen inerten, hochporösen Feststoff mit einer spezifischen Oberfläche nach BET von 900 bis 1800 $m^2/g$, bestimmt durch Argonadsorption bei $-196°C$, oder pyrogenes Siliciumdioxid mit einer spezifischen Oberfläche nach BET von 50 bis 500 $m^2/g$, bestimmt durch Argonadsorption bei $-196°C$, leitet, wobei die Oberfläche der Feststoffe mit 0,1 bis 5 Gew.-% Aluminium, Zink, Chrom oder

Eisen, bezogen auf das Gewicht des Feststoffs mit einer spezifischen Oberfläche von 1000 $m^2/g$, belegt ist und wobei nur die Schwefel- bzw. Mercaptanverbindungen katalytisch zerlegt und adsorbiert und als nichtriechende Bruchteile desorbiert werden.

Gemäss einer bevorzugten Ausführungsform verwendet man als Feststoff Aktivkohle, Zeolithe oder Metalloxide, insbesondere Aluminiumoxid oder Siliciumdioxid. Gemäss einer anderen bevorzugten Ausführungsform besitzt die Aktivkohle eine spezifische Oberfläche nach BET von 1000 bis 1800 $m^2/g$, deren Oberfläche mit 0,1 bis 5 Gew.-% Aluminium, Zink, Chrom oder Eisen, bezogen auf das Gewicht der Aktivkohle mit einer spezifischen Oberfläche von 1000 $m^2/g$, belegt ist, oder man verwendet ein Gemisch solcher Aktivkohlen.

Gegenstand der Erfindung ist weiterhin ein Verfahren zur Herstellung eines hochporösen Feststoffs mit einer spezifischen Oberfläche nach BET von 900 bis 1800 $m^2/g$, bestimmt durch Argonadsorption bei −196°C, oder Verfahren zur Herstellung von pyrogenem Siliciumdioxid mit einer spezifischen Oberfläche nach BET von 50 bis 500 $m^2/g$, bestimmt durch Argonadsorption bei −196°C, wobei die Oberfläche der Feststoffe mit 0,1 bis 5 Gew.-% Aluminium, Zink, Chrom oder Eisen, bezogen auf das Gewicht des Feststoffs mit einer spezifischen Oberfläche von 1000 $m^2/g$, belegt ist, das dadurch gekennzeichnet ist, dass man den Feststoff oder das pyrogene Siliciumdioxid mit entmineralisiertem Wasser wäscht, anschliessend bei einer Temperatur von 150 bis 300°C im Vakuum bei $10^{-4}$ bis $10^{-7}$ bar während einer Zeit von 30 min bis 10 h belässt und dann zu dem Feststoff im Vakuum unter Rühren eine Lösung eines geeigneten Metallsalzes von Aluminium, Zink, Chrom oder Eisen zugibt, den unter Dampf befindlichen Feststoff langsam mindestens jedoch während einer Zeit von 1 h, auf Zimmertemperatur abkühlt und ihn anschliessend trocknet.

Gemäss bevorzugten Ausführungsformen der Erfindung verwendet man als Lösung eine wässerige Lösung eines wasserlöslichen Metallsalzes, insbesondere eines Chlorids, Chromats oder Titanats, und/oder man wäscht den Feststoff zur Verhandlung in einem Ultraschallbad.

Die Erfindung betrifft weiterhin ein Verfahren zur Herstellung einer hochporösen Aktivkohle mit einer spezifischen Oberfläche nach BET von 1000 bis 1800 $m^2/g$, bestimmt durch Argonadsorption bei −196°C, die auf ihrer Oberfläche mit 0,1 bis 5 Gew.-% Aluminium, Zink, Chrom oder Eisen, bezogen auf das Gewicht der Aktivkohle mit einer spezifischen Oberfläche von 1000 $m^2/g$, belegt ist, das dadurch gekennzeichnet ist, dass man die Aktivkohle mit entmineralisiertem Wasser wäscht, sie anschliessend bei einer Temperatur von 150 bis 300°C im Vakuum bei $10^{-4}$ bis $10^{-7}$ bar während einer Zeit von 30 min bis 10 h belässt und dann im Vakuum zu der Kohle unter Rühren eine Lösung eines geeigneten Metallsalzes zugibt, wobei gegebenenfalls gekühlt wird, und die unter Dampf befindliche Kohle langsam, mindestens jedoch während einer Zeit von 1 h, auf Zimmertemperatur abkühlt und sie anschliessend trocknet.

Gemäss einer bevorzugten erfindungsgemässen Ausführungsform verwendet man eine wässerige Lösung eines wasserlöslichen Metallsalzes, insbesondere eines Chlorids, Chromats oder Titanats.

Die Erfindung betrifft weiterhin ein hochspezifisches Adsorbens zur Durchführung des erfindungsgemässen Verfahrens zur Adsorption und katalytischen Zerlegung übelriechender Darmgase, welches Adsorbens dadurch gekennzeichnet ist, dass es 0 bis 50% hochporösen, pulverförmigen Feststoff, der mit 0,1 bis 5 Gew.-% Aluminium belegt ist, 0 bis 50 Gew.-% hochporösen, pulverförmigen Feststoff, der mit 0,1 bis 5 Gew.-% Zink belegt ist, und 0 bis 50 Gew.-% hochporösen Feststoff, der mit 0,1 bis 5 Gew.-% Chrom belegt ist, im Gemisch enthält, wobei der hochporöse Feststoff aus der Gruppe ausgewählt wird: Aktivkohle, Zeolithe, Metalloxide, insbesondere Aluminiumoxid oder Siliciumdioxid, mit einer spezifischen Oberfläche nach BET, bestimmt durch Argonadsorption bei −196°C, von 900 bis 1800 $m^2/g$ oder pyrogenem Siliciumdioxid mit einer spezifischen Oberfläche zwischen 50 und 500 $m^2/g$, bestimmt durch Argonadsorption bei −196°C, wobei sich die Gewichtsprozentangaben jeweils auf das Gewicht des Feststoffs mit einer spezifischen Oberfläche von 1000 $m^2/g$ beziehen.

Gemäss bevorzugten erfindungsgemässen Ausführungsformen enthält das hochspezifische Adsorbens als hochporösen Feststoff Aktivkohle, insbesondere 0 bis 50% Aktivkohle, die mit 0,15 bis 2,5 Gew.-% Aluminium belegt ist, 0 bis 50% Aktivkohle, die mit 0,25 bis 3,5 Gew.-% Zink belegt ist, und 0 bis 50% Aktivkohle, die mit 0,3 bis 5 Gew.-% Chrom belegt ist, im Gemisch. Gemäss einer weiteren bevorzugten Ausführungsform der Erfindung enthält das hochspezifische Adsorbens die mit Aluminium belegte Aktivkohle, die mit Zink belegte Aktivkohle und die mit Chrom belegte Aktivkohle im Verhältnis 1:1:1.

Die Anmelderin hat überraschenderweise gefunden, dass die Adsorptionsfähigkeit von bestimmten inerten, hochporösen Feststoffen bei der Adsorption der übelriechenden Bestandteile aus Darmgasen wesentlich verbessert werden kann, wenn man die Oberfläche der hochporösen Feststoffe mit ausgewählten Metallen belegt. Durch Modifizierung der Oberfläche der hochporösen Feststoffe mit Metallen wird die Adsorptionsfähigkeit für Thiole vergrössert und die adsorbierte Thiolmenge erhöht.

Das beanspruchte Verfahren und Mittel zur Adsorption und katalytischen Zerlegung übelriechender Darmgase unterscheidet sich von dem aus der GB-A Nr. 1235874 bekannten Stand der Technik dadurch, dass die verwendete Aktivkohle nicht nur mit bis zu 15 Gew.-% Metallsalzen belegt ist, sondern dass die Aktivkohle im beanspruchten Verfahren nach einem Waschprozess im Vakuum bei $10^{-3}$ bis $10^{-6}$ mbar bei Temperaturen zwischen 100°C und 500°C einmal von adsorbierten Substanzen befreit wird und zum anderen bestimmte aktive Endgruppen, wie zum Beispiel OH- und

COOH-Gruppen, zu einem bestimmten Teil von der Aktivkohleoberfläche entfernt werden.

Bei bestehendem Vakuum wird dann die Metallsalzlösung zugegeben, wobei eine feste Bindung zwischen Metallion und Aktivkohleoberflächen radikal entsteht. Dabei werden derselben Aktivkohle nacheinander eine Kombination verschiedener Metalle angeboten (zum Beispiel Al, Zn, Cr).

Erfindungsgemäss wird die Aktivkohle in einer geeigneten Oberflächendichte mit Metallionen belegt, und die Belegung mit Metallionen wird im Vakuum nach genauer Radikalisierung der Aktivkohleoberfläche bei geeigneter Temperatur durchgeführt.

Die bei dem erfindungsgemässen Verfahren verwendeten, hochspezifischen Adsorbenzien werden beispielsweise in Filterpatronen in den Kolostomiebeutel eingebracht, und überraschenderweise zeigte es sich, dass im allgemeinen 0,5 bis 3 g an Adsorbens ausreichen, um eine 12- bis 24stündige Arbeitszeit des Filters am Kolostomiepatienten zu gewährleisten. Bei dem erfindungsgemässen Verfahren und durch das erfindungsgemässe Mittel werden weiterhin alle im Darm vorkommenden, übelriechenden und giftigen Substanzen entfernt bzw. unschädlich gemacht. Ausserdem wird die Adsorptionsoberfläche durch die auftretenden Schwefelverbindungen nicht vergiftet und behält, wie oben angegeben, ihre Wirkung während langer Zeit bei.

Erfindungsgemäss wird ein hochspezifisches Adsorbens verwendet, dessen Oberfläche gezielt modifiziert wird. Das erfindungsgemässe verwendete Adsorbens muss ein inerter, hochporöser Feststoff sein, d.h. ein Feststoff, der eine grosse spezifische Oberfläche sowie eine Porenverteilung, die von Mikroporen über Mesoporen zu Makroporen reicht, aufweist. Der Feststoff kann in verschiedenen Korngrössenbereichen vorliegen. Er kann z.B. ein Pulver sein oder als Pulverpressling in Zylinderform oder Kugelform vorliegen. Die Korngrössen des Feststoffs liegen im allgemeinen im Bereich von 0,1 bis 3 mm, vorzugsweise bei 0,2 bis 0,5 mm.

Hochporöse Feststoffe, die erfindungsgemäss beispielsweise verwendet werden können, sind Aktivkohle, Zeolithe, Metalloxide, wie z.B. Aluminiumoxid und Siliciumdioxid, wobei letzteres als Gel oder als pyrogenes, porenfreies Siliciumdioxid eingesetzt werden kann.

Der erfindungsgemäss verwendete, poröse Feststoff, nämlich die Aktivkohle, die Zeolithe, die Metalloxide, wie Aluminiumoxid oder Siliciumdioxid, muss weiterhin eine grosse spezifische Oberfläche nach BET von 900 bis 1800 $m^2/g$, vorzugsweise von 1000 bis 1800 $m^2/kg$, aufweisen. Je grösser die Oberfläche ist und je mehr Adsorptionszentren mit vorwiegend polarer Wechselwirkung vorhanden sind, um so besser ist der hochporöse Feststoff für die vorliegende Erfindung geeignet. Die Oberfläche wird nach dem bekannten BET-Verfahren durch Argonadsorption bei −196°C bestimmt.

Als Feststoff kann man bei der vorliegenden Erfindung auch pyrogenes, porenfreies Siliciumdioxid verwenden, das eine BET-Oberfläche von 50 bis 500 $m^2/g$, vorzugsweise von 400 bis 500 $m^2/g$, aufweist. Auch für diesen Feststoff gilt, je grösser die Oberfläche ist und je mehr Adsorptionszentren mit vorwiegend polarer Nachwirkung vorhanden sind, um so besser ist der Stoff geeignet.

Bei der vorliegenden Erfindung wird bevorzugt Aktivkohle verwendet. Solche Aktivkohlen sind im Handel erhältlich. Aktivkohlen sind hochporöse Substanzen mit spezifischen Oberflächen von 600 bis 1000 $m^2/g$. Sie besitzen gute Adsorptionseigenschaften sowohl für polare Substanzen, wie Wasser und Alkohole, wie auch für unpolare Substanzen, wie Benzol oder Alkane, d.h. im wesentlichen besitzen die Aktivkohlen unspezifische Adsorbenseigenschaften.

Einer gezielten Modifizierung der Adsorbensoberfläche, vorzugsweise der Aktivkohleoberfläche, muss eine genaue Charakterisierung der Adsorbensoberfläche bzw. Aktivkohleoberfläche vorausgehen. Ergebnisse dieser Charakterisierung sind die Art, die Anzahl, die Energetik und die bevorzugte Wechselwirkung der Adsorptionsplätze der Adsorbens- bzw. Aktivkohleoberfläche.

Aktivkohle enthält auf ihrer inneren und äusseren Oberfläche bestimmt aktive Endgruppen, wie z.B. $-OH$-, $-COOH$-, $-CO_2$- und CO-Gruppen, wie es im folgenden dargestellt wird.

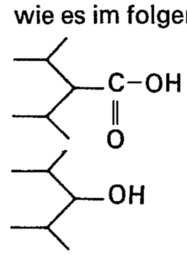

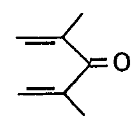

Die Anzahl und Dichte und Art der aktiven Endgruppen, die die Adsorptionsplätze auf der Aktivkohleoberfläche ausbilden, können beispielsweise im Vakuum, wie in der folgenden Gleichung dargestellt, variiert werden.

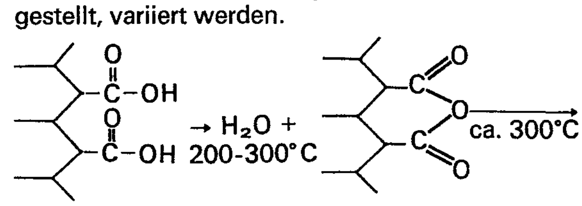


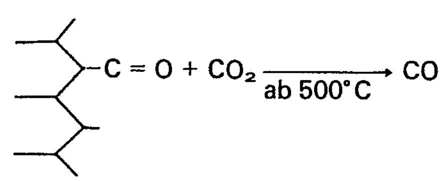


Die Variation der aktiven Endgruppen ist u.a. von der Art der verwendeten Ausgangskohle, der vorhandenen Endgruppen, der Höhe des Vakuums und der Temperatur abhängig.

Zeolithe, Siliciumdioxid und Aluminiumoxid enthalten auf den Oberflächen als Adsorptionsplätze OH-Gruppen, die basisch oder sauer bzw. basisch und sauer oder die, wie bei Siliciumdioxid, neutral reagieren. Diese können paarweise oder singulär auftreten.

Die Variation der aktiven Endgruppen ist für eine Ankopplung von Katalysatormolekülen und deren Oberflächendichtevariation wichtig.

Die Charakterisierung der Oberfläche des porösen Feststoffs geschieht mit Adsorptions-, Desorptions- und mit photoelektronen-spektroskopischen Methoden. Als Kenngrössen erhält man Art, Anzahl, Verteilung und Energetik der Adsorptionsplätze auf der Oberfläche des porösen Feststoffs (vgl. G. Hellwig, „Farbe und Lack", *81*, S. 705 [1975]; G. Hellwig, *ibid.*, *82*, S. 582 [1976]; G. Hellwig, „Blücher Manual", Stand Juli 1976, S. 8 [1976]).

Stark vereinfacht lassen sich die Adsorptionsplätze in zwei Gruppen unterteilen, in solche, die hauptsächlich mit polaren Molekülen wechselwirken, und in solche, die hauptsächlich mit unpolaren Molekülen wechselwirken. Schwierigkeiten werden immer dann auftreten, wenn aus polaren Trägergasgemischen oder Flüssigkeiten bestimmte polare Substanzen bzw. aus unpolaren Trägergasgemischen oder Flüssigkeiten unpolare Substanzen adsorptiv entfernt werden müssen. Hier werden immer die polaren Trägergas- oder Lösungsmittelmoleküle bzw. die unpolaren Trägergas- oder Lösungsmittelmoleküle die entsprechenden Adsorptionsplätze des porösen Feststoffs besetzen und somit das Adsorbens für seine eigentliche Aufgabe schnell unbrauchbar machen.

Beim Filtereinsatz im Darmbereich und Darmausgang müssen polare Substanzen aus polaren Lösungsmitteln und polaren Trägergasgemischen entfernt werden. Um die Arbeitszeitdauer eines Filters für die oben beschriebene Aufgabe zu vergrössern, wird die charakterisierte Adsorbensoberfläche mit geeigneten Metallsalzen in geeigneter Oberflächendichte modifiziert. Dabei wird die Adsorbensoberfläche mit Metallen belegt. Die Belegung der Adsorbensoberfläche muss auf spezifische Weise geschehen. Durch eine gezielte Vorbehandlung des Adsorbens wird eine bestimmte Zahl von verfügbaren Adsorptionsplätzen auf der Adsorbensoberfläche geschaffen und eine gewisse Anzahl dieser verfügbaren Plätze wird dann mit Metallsalzen belegt.

Die Auswahl der Metallsalze richtet sich nach den physikalischen Eigenschaften der zu entfernenden Substanzen. Die Metallsalze werden in einem Testverfahren ermittelt. Die Oberflächendichte richtet sich nach Grösse und Dipolmoment der zu entfernenden Substanzen.

Erfindungsgemäss werden die Adsorbensoberflächen und insbesondere die Aktivkohleoberflächen mit Metallen in geeigneter Oberflächendichte belegt.

Als Metalle verwendet man Aluminium, Zink, Chrom oder Eisen. Aluminium, Zink und Chrom sind besonders bevorzugt.

Die Adsorbens- und insbesondere die Aktivkohleoberfläche wird mit dem Metall in einer Menge von 0,1 bis 5 Gew.-%, bezogen auf 1 g Adsorbens, mit einer spezifischen Oberfläche von 1000 $m^2/g$ belegt. Vorzugsweise erfolgt die Belegung in einer Menge von 0,1 bis 3 Gew.-%, am meisten bevorzugt in einer Menge von 0,2 bis 2,0 Gew.-%.

Bevorzugte Belegungskonzentrationen für Aluminium liegen im Bereich von 0,15 bis 2,5 Gew.-%, für Zink im Bereich von 0,25 bis 3,5 Gew.-% und für Chrom im Bereich von 0,3 bis 5,0 Gew.-%. Besonders bevorzugt verwendet man als Adsorbens Aktivkohle, die mit den obengenannten Metallen, Aluminium, Zink und Chrom, in den angegebenen Bereichen belegt ist.

Die Belegung der Metalle ist als Gewichtsprozent, bezogen auf das Adsorbens mit einer spezifischen Oberfläche von 1000 $m^2/g$, angegeben. Dies bedeutet, dass die Belegung der Metalle von der Oberfläche abhängt und dass man beispielsweise für die Belegung eines porösen Feststoffs, der nur eine Oberfläche von 500 $m^2/g$ aufweist, die Hälfte der oben aufgeführten Gewichtsprozente benötigt, andererseits für einen Feststoff, der eine Oberfläche von beispielsweise 1500 $m^2/g$ besitzt, drei Halbe der oben aufgeführten Gewichtsprozent an Metallen benötigt.

Die Belegung des Adsorbens, insbesondere der Kohle, wird im allgemeinen durchgeführt, indem man das Adsorbens nach einer Vorbehandlung mit einer wässerigen Lösung eines wasserlöslichen Salzes des zu belegenden Metalls oder, sofern die verwendeten Salze wasserunlöslich sind, mit einer geeigneten Lösung in einem organischen Lösungsmittel behandelt.

Als Salze kann man z.B. die Chloride, Chromate, Titanate usw. verwenden. Man verwendet vorzugsweise wasserlösliche Salze, z.B. Zinkchlorid oder Chromtrichlorid oder Eisen(III)chlorid. Sind die Salze, wie z.B. Aluminiumtrichlorid, nicht in Wasser löslich, so kann man als Lösungsmittel wasserfreie Alkohole, wie z.B. Äthanol, verwenden.

Zur Vorbehandlung wird das Adsorbens in entmineralisiertem Wasser gewaschen. Das Waschen erfolgt, um feinste Staubteilchen und möglicherweise auf der Oberfläche des Adsorbens haftende Fremdstoffe zu entfernen. Das Waschen mit Wasser kann durchgeführt werden, indem man das Adsorbens zu der 3- bis 10fachen Wassermenge gibt und es während einer Zeit von 30 min bis 3 h rührt oder es beispielsweise im Ultraschallbad während einer Zeit von 30 bis 90 min bei 33 kHz und 1 kW Schalleistung wäscht. Diese Waschbedingungen sind nicht kritisch und können auf geeignete Weise variiert werden.

Danach wird das Adsorbens in einem Reaktionsgefäss der erforderlichen Vorbehandlung unterworfen. Bei dieser Vorbehandlung wird die Adsorbensoberfläche so modifiziert, dass ausreichend Adsorptionsplätze an der Oberfläche vorhanden sind.

Zur Vorbehandlung wird auf eine Temperatur im Bereich von 100 bis 500°C und einem Druck im Bereich von $1 \times 10^{-4}$ bis $1 \times 10^{-7}$ bar erhitzt. Bevorzugte Temperaturen für Aktivkohle liegen im Bereich von 150 bis 300°C, für Zeolithe und Siliciumdioxid im Bereich von 200 bis 400°C und für Aluminiumoxid im Bereich von 250 bis 350°C bei einem Druck im Bereich von $1 \times 10^{-4}$ bis $1 \times$

$10^{-7}$ bar. Die geeigneten Reaktionsbedingungen können leicht vom Fachmann bestimmt werden.

Verwendet man beispielsweise bei Aktivkohle eine Temperatur von 150 bis 300°C und einen Druck von $5 \times 10^{-6}$ bar, so existieren an der Aktivkohleoberfläche ca. $5 \times 10^{14}$ Adsorptionsplätze/$cm^2$. Verwendet man eine Aktivkohle mit einer spezifischen Oberfläche nach BET (Argonadsorption bei −196°C) von ca. 1000 $m^2$/g, so folgt, dass 1 g Aktivkohle $5 \times 10^{21}$ Adsorptionsplätze der Art −OH und −COO besitzt.

Als günstigste Belegung mit $AlCl_3$ für die Thioladsorption hat sich eine Menge von 111 mg $AlCl_3$/g Aktivkohle experimentell herausgestellt. Das heisst, im statistischen Mittel ist jeder zehnte Adsorptionsplatz mit einem Al-Atom besetzt. Neueste Messungen zeigten, dass auch bereits 11 mg $AlCl_3$/g Aktivkohle eine ähnlich gute Wirkung bei der Methanthiolvernichtung erbringen. Für die Vernichtung von Thioessigsäure bzw. Schwefelkohlenstoff wurden pro Gramm Aktivkohle 13 bis 130 mg $ZnCl_2$ bzw. 15 bis 150 mg $CrCl_3$ benötigt.

Die Chloride des Zinks und Chroms sind wasserlöslich. Aluminiumtrichlorid wird in wasserfreien Lösungsmitteln, z.B. Alkoholen, gelöst. Die Lösung wird nach der Temperatur-Vakuumbehandlung direkt zu dem im Vakuum gerührten Adsorbens, vorzugsweise zu der Kohle, durch eine Spezialflüssigkeitszufuhreinrichtung gegeben. Dabei verringert sich der Druck im Reaktionsgefäss auf ca. 40 bar. Die jetzt unter Lösungsmittel, z.B. Wasserdampf, stehenden Adsorbenzien verbleiben im Reaktionsgefäss bis zur Abkühlung, mindestens jedoch 1, vorzugsweise 4 h. Danach wird gelüftet und das Wasser und noch vorhandenes Lösungsmittel abgetrocknet. Das jetzt mit Metall belegte Adsorbens ist für den Gebrauch fertig.

Erfindungsgemäss kann man auch Gemische aus Adsorbenzien verwenden, die mit unterschiedlichen Metallen belegt sind. Bei einer bevorzugten Ausführungsform verwendet man ein Gemisch aus verschiedenen Aktivkohlen, die mit Aluminium, Zink und Chrom belegt sind.

Zur Prüfung des erfindungsgemässen hochspezifischen Adsorbens wird eine Reihe von Aktivkohlen mit hohem Anteil an Adsorptionsplätzen mit vorwiegend polarer Wechselwirkung mit verschiedenen Katalysatoren belegt. So wurden je 3 Mengen von 10 g Aktivkohle des Types Norit®-Azo mit den Chloriden von Aluminium, Chrom, Eisen und Zink belegt. Die Adsorptionswirksamkeit dieser modifizierten Aktivkohle jeweils gegen Methanthiol ($CH_3SH$), Äthanthiol ($C_2H_5SH$), Thioessigsäure ($CH_3COSH$) und Schwefelkohlenstoff ($CS_2$) wurde in Gegenwart von Wasser, $CO_2$ und Methan getestet.

Als Adsorptiv wurde ein Modelldarmgas verwendet. Es bestand aus einem mit Wasser und der Schwefelverbindung gesättigten Kohlendioxidträgergas. Die Transportgeschwindigkeit $V_T$ des zu filtrierenden Schwefelkohlenstoffs variierte von 0,17 bis 1,8 ml/s.

Hierzu wurde eine Apparatur konstruiert, die in einem Vakuumrezipienten eine elektronische Mikrowaage, ein Kalorimeter und ein Massenspektrometer enthält. Über ein Dosierventil wird das durch das Filter aus modifizierter Aktivkohle strömende Modelldarmgas mit obiger Zusammensetzung in die Messapparatur eingelassen. Den schematischen Aufbau der Messapparatur zeigt Fig. 1 für ein Gasgemisch aus $CO_2$, $H_2O$ und $CS_2$ bzw. $CH_3SH$, $C_2H_5SH$ und $CH_3COSH$.

Bei einer konstanten Temperatur von 37°C wird das mit konstanter Geschwindigkeit strömende Kohlendioxidgas zunächst mit $H_2O$ und danach mit $CS_2$ bzw. $CH_3SH$, $C_2H_5SH$ und $CH_3COSH$ gesättigt. Das so entstandene Modelldarmgas durchströmt das zu testende Kohlefilter und gelangt gefiltert in einen Rezipienten. In diesem Rezipienten werden gleichzeitig an der gleichen Kohle wie im Filter Adsorptionseigenschaften, wie adsorbierte Menge, freiwerdende Adsorptionsenergie und Adsorptionsenergetik, sowie die gefilterte $CS_2$- bzw. $CH_3SH$-, $C_2H_5SH$- und $CH_3COSH$-Menge gemessen. Gleichzeitig mit diesen Messungen wird von einem Massenspektrometer ständig die Zusammensetzung des Modellgases bestimmt, so dass die Filterwirkung während des gesamten Filtervorgangs bis zum Durchbruch und auch danach bekannt ist.

Aus diesen Versuchen folgt, dass die mit Metallen beladene Aktivkohle für Darmgase eine hohe Adsorption zeigt und dass von den getesteten Metallsalzen das Zinkchlorid die besten Ergebnisse liefert. 1 g mit obigen Metallen belegte Kohle vernichtet 2 bis 3 ml $CH_3SH$, $C_2H_5SH$, $CH_3COSH$ und $CS_2$.

Weitere Versuche haben gezeigt, dass die anderen, oben erwähnten, mit den Metallen belegten Adsorbenzien ähnlich gute Wirkungen zeigen.

Es wurde weiterhin gefunden, dass für jedes der zu entfernenden Gase (Methanthiol, Äthanthiol, Thioessigsäure und Schwefelkohlenstoff) ein anderes, speziell vorbehandeltes Adsorbens benötigt wurde. Für Methan- und Äthanthiol zeigte sich Aluminium bzw. Chrom als am geeignetsten. Für Thioessigsäure ist dies Zink und für Schwefelkohlenstoff Chrom. Daraus folgt, dass das am besten wirkende Kolostomiefilter eine Mischung aus drei verschieden modifizierten Adsorbenzien, vorzugsweise Kohlen, enthält. Erfindungsgemäss verwendet man daher bevorzugt Gemische aus Adsorbenzien, besonders bevorzugt Aktivkohlen, die mit unterschiedlichen Metallen belegt sind.

Bevorzugt verwendet man erfindungsgemäss als Adsorbens ein Gemisch, das 0 bis 50, vorzugsweise 20 bis 40, am meisten bevorzugt 33,33% Adsorbens, besonders bevorzugt Aktivkohle, das mit 0,1 bis 5, vorzugsweise 0,15 bis 2,5 Gew.-% Al belegt ist, 0 bis 50%, vorzugsweise 20 bis 40%, am meisten bevorzugt 33,33% Adsorbens, vorzugsweise Aktivkohle, das mit 0,1 bis 5, vorzugsweise 0,25 bis 3,5% Zink belegt ist, und 0 bis 50%, vorzugsweise 20 bis 40%, am meisten bevorzugt 33,33% Adsorbens, vorzugsweise Aktivkohle, das mit 0,1 bis 5, vorzugsweise 0,3 bis 5% Chrom belegt ist, enthält. Überraschenderweise zeigte es sich, dass 0,5 bis 3 g eines solchen Gemisches während einer Zeit von mindestens 12 h für die

Adsorption übelriechender Darmgase wirksam sind.

Das oben erwähnte erfindungsgemässe Adsorbens wird in eine zylindrische Filterpatrone oder einen kissenartigen Beutel eingesetzt, wobei die Patrone oder der Beutel in den Kolostomiebeutel eingesteckt wird.

Die folgenden Beispiele erläutern die Erfindung.

*Beispiel 1:*

100 g Aktivkohle werden in 3000 ml entmineralisiertem Wasser unter Rühren 30 min gewaschen. Danach wird die überstehende Suspension abdekantiert und die Kohle in einen heiz- und kühlbaren Vakuumrezipienten aus Edelstahl eingebracht. Unter gleichzeitigem Abpumpen bis $10^{-6}$ bar und Erhitzen auf 150°C während 60 min werden noch adsorbierte Fremdstoffe desorbiert.

1,3 g Zinkchlorid (p.a.) werden in 300 ml destilliertem Wasser gelöst. Die Lösung wird zu der auf 100°C abgekühlten Kohle bei $10^{-5}$ bar unter starkem Rühren durch eine Spezialvakuumzufuhrleitung zugegeben. Im Inneren des Rezipienten stellt sich sofort ein Druck von 40 Torr ein. Die Kohle wird ca. 4 h stehengelassen und dann wird entlüftet und die Kohle wird in einem normalen Trockenschrank getrocknet.

*Beispiel 2:*

50 g Aktivkohle werden in 200 ml entmineralisiertem Wasser bei Zimmertemperatur im Ultraschallbad 60 min bei 33 kHz und 1 kW Schalleistung gewaschen.

Aus der Ultraschallwanne wird die Flüssigkeit abdekantiert und die gewaschene Kohle wird in ein Reaktionsgefäss aus Stahl gegeben. Das Reaktionsgefäss wird im Verlauf von 30 min auf 200°C erhitzt. Der Druck wird gleichzeitig auf $10^{-6}$ bar erniedrigt. Während 30 bis 120 min und bei $10^{-6}$ bar werden die adsorbierten Fremdstoffe entfernt.

Getrennt werden 0,55 g Aluminiumtrichlorid in 200 ml reinem, wasserfreiem Äthanol gelöst. Diese Lösung wird zu der auf 120°C abgekühlten Kohle bei einem Druck von $10^{-6}$ bar unter starkem Rühren zugegeben. Die Kohle wird noch 2 h weitergerührt, anschliessend wird entlüftet und die Kohle im üblichen Trockenschrank getrocknet.

*Beispiel 3:*

200 g Aktivkohle werden in 4000 ml entmineralisiertem Wasser unter Rühren 60 min bei Zimmertemperatur gewaschen. Danach wird die überstehende Suspension abdekantiert und die Kohle 2 h bei 220°C und $10^{-6}$ bar zur Desorption noch adsorbierter Fremdstoffe erhitzt.

Getrennt werden 1,5 g Chromtrichlorid in 300 ml bidestilliertem Wasser gelöst. Die Lösung wird zu der auf 80°C abgekühlten Kohle bei $10^{-6}$ bar unter starkem Rühren zugegeben. Die Kohle wird eine weitere Stunde gerührt. Anschliessend wird der Kolben entlüftet und die Kohle wird im Vakuum getrocknet.

*Beispiel 4:*

100 g Aktivkohlepulver (Chemviron Sc. XII, 6×12, spezifische Oberfläche ca. 1000 $m^2/g$, mit ca. $5 \times 10^{14}$ Adsorptionsplätzen pro Quadratzentimeter mit vorwiegend polarer Wechselwirkung, mit einer Adsorptionswärme gegen Wasser von 20,2 kJ/mol) werden in einer Ultraschallwaschanlage mit 1 kW Leistung bei 30 kHz 0,5 h in 500 $cm^3$ entmineralisiertem Wasser gewaschen und so von groben Verunreinigungen und Feinstpartikeln (Stäuben) befreit. Die Kohle wird luftgetrocknet.

In einem evakuierbaren Behälter wird die trokkene Kohle bei einem Druck von $10^{-1}$ bis $10^{-3}$ mbar und einer Temperatur von 150°C 0,5 h gerührt. Nach Erniedrigen der Temperatur von 150 auf 100°C wird in den evakuierten Behälter zu der Kohle unter Rühren 250 ml $FeCl_3$-Lösung mit 1,5 g $FeCl_3$ in je 250 ml bidestilliertem Wasser zugegeben.

Unter Rühren verweilt das Gemisch Kohle/Salz-Lösung im Behälter ca. 0,5 h bis die Temperatur auf ca. 40°C gesunken ist. Danach wird die flüssige Phase abgefiltert. Die verbleibende modifizierte Aktivkohle wird danach so lange in mehrmals (bis zu dreimal) zu wechselnden 500 ml Wasserbädern ultraschallgewaschen bis die Kohle kein Eisen mehr in die wässerige Phase abgibt (Nachweisgrenze 0,5 ppm).

Danach wird die Kohle luftgetrocknet. Während der ganzen Prozedur blieb die Korngrössenverteilung der Aktivkohle erhalten.

*Beispiel 5:*

100 g Aktivkohlepulver (Chemviron Sc. XII, 6×12, spezifische Oberfläche ca. 1000 $m^2/g$, mit ca. $5 \times 10^{14}$ Adsorptionsplätzen pro Quadratzentimeter mit vorwiegend polarer Wechselwirkung, mit einer Adsorptionswärme gegen Wasser von 20,2 kJ/mol) werden in einer Ultraschallwaschanlage mit 1 kW Leistung bei 30 kHz 0,5 h 500 $cm^3$ entmineralisiertem Wasser gewaschen und so von groben Verunreinigungen und Feinstpartikeln befreit. Danach wird die Kohle luftgetrocknet.

In einem evakuierbaren Behälter wird die trokkene Kohle bei einem Druck von $10^{-1}$ bis $10^{-3}$ mbar und einer Temperatur von 300°C 0,5 h gerührt. Nach Erniedrigen der Temperatur auf 100°C wird in den die Kohle enthaltenden evakuierten Behälter 250 ml $ZnNO_3$-Lösung mit 1,3 g $ZnNO_3$ in 250 ml bidestilliertem Wasser unter Rühren zugegeben. Danach wird wie in Beispiel 4 verfahren.

*Beispiel 6:*

Jeweils 33,33 g der bei den Beispielen 1, 2 und 3 erhaltenen Kohlen werden auf einer Analysenwaage genau abgewogen. Die Kohlen werden in einem Mörser gut vermischt und dann wird jeweils 1 g in Patronen abgepackt. Man erhält 100 Patronen, die in Kolostomiebeutel eingesetzt werden. Es zeigt sich, dass die Patronen während einer Zeit

von mehr als 12 h alle übelriechenden Darmgase adsorbieren.

## Patentansprüche

1. Verfahren zur Adsorption und katalytischen Zerlegung übelriechender, Wasserdampf, Methanol, Kohlenstoffmonoxid und Kohlenstoffdioxid und verschiedene schwefelhaltige Gase, insbesondere Mercaptane, enthaltender Darmgase, dadurch gekennzeichnet, dass man die Darmgase durch einen inerten, hochporösen Feststoff mit einer spezifischen Oberfläche nach BET von 900 bis 1800 $m^2/g$, bestimmt durch Argonadsorption bei −196°C, oder pyrogenes Siliciumdioxid mit einer spezifischen Oberfläche nach BET von 50 bis 500 $m^2/g$, bestimmt durch Argonadsorption bei −196°C, leitet, wobei die Oberfläche der Feststoffe mit 0,1 bis 5 Gew.-% Aluminium, Zink, Chrom oder Eisen, bezogen auf das Gewicht des Feststoffs mit einer spezifischen Oberfläche von 1000 $m^2/g$, belegt ist und wobei nur die Schwefel- bzw. Mercaptanverbindungen katalytisch zerlegt und adsorbiert und als nichtriechende Bruchteile desorbiert werden.

2. Verfahren nach Anspruch 1, dadurch gekennzeichnet, dass man als Feststoff Aktivkohle, Zeolithe oder Metalloxide, insbesondere Aluminiumoxid oder Siliciumdioxid, verwendet.

3. Verfahren nach einem der beiden Ansprüche 1 oder 2, dadurch gekennzeichnet, dass man Aktivkohle mit einer spezifischen Oberfläche nach BET von 1000 bis 1800 $m^2/g$, deren Oberfläche mit 0,1 bis 5 Gew.-% Aluminium, Zink, Chrom oder Eisen, bezogen auf das Gewicht der Aktivkohle mit einer spezifischen Oberfläche von 1000 $m^2/g$, belegt ist, oder ein Gemisch solcher Aktivkohlen verwendet.

4. Verfahren zur Herstellung eines hochporösen Feststoffs mit einer spezifischen Oberfläche nach BET von 900 bis 1800 $m^2/g$, bestimmt durch Argonadsorption bei −196°C, oder Verfahren zur Herstellung von pyrogenem Siliciumdioxid mit einer spezifischen Oberfläche nach BET von 50 bis 500 $m^2/g$, bestimmt durch Argonadsorption bei −196°C, wobei die Oberfläche der Feststoffe mit 0,1 bis 5 Gew.-% Aluminium, Zink, Chrom oder Eisen, bezogen auf das Gewicht des Feststoffs mit einer spezifischen Oberfläche von 1000 $m^2/g$, belegt ist, dadurch gekennzeichnet, dass man den Feststoff oder das pyrogene Siliciumdioxid mit entmineralisiertem Wasser wäscht, anschliessend bei einer Temperatur von 150 bis 300°C im Vakuum bei $10^{-4}$ bis $10^{-7}$ bar während einer Zeit von 30 min bis 10 h belässt und dann zu dem Feststoff im Vakuum unter Rühren eine Lösung eines geeigneten Metallsalzes von Aluminium, Zink, Chrom oder Eisen zugibt, den unter Dampf befindlichen Feststoff langsam, mindestens jedoch während einer Zeit von 1 h, auf Zimmertemperatur abkühlt und ihn anschliessend trocknet.

5. Verfahren nach Anspruch 4, dadurch gekennzeichnet, dass man als Lösung eine wässerige Lösung eines wasserlöslichen Metallsalzes, insbesondere eines Chlorids, Chromats oder Titanats, verwendet.

6. Verfahren nach einem der beiden Ansprüche 4 oder 5, dadurch gekennzeichnet, dass man den Feststoff zur Vorbehandlung in einem Ultraschallbad wäscht.

7. Verfahren zur Herstellung einer hochporösen Aktivkohle mit einer spezifischen Oberfläche nach BET von 1000 bis 1800 $m^2/g$, bestimmt durch Argonadsorption bei −196°C, die auf ihrer Oberfläche mit 0,1 bis 5 Gew.-% Aluminium, Zink, Chrom oder Eisen, bezogen auf das Gewicht der Aktivkohle mit einer spezifischen Oberfläche von 1000 $m^2/g$, belegt ist, dadurch gekennzeichnet, dass man die Aktivkohle mit entmineralisiertem Wasser wäscht, sie anschliessend bei einer Temperatur von 150 bis 300°C im Vakuum bei $10^{-4}$ bis $10^{-7}$ bar während einer Zeit von 30 min bis 10 h belässt und dann im Vakuum zu der Kohle unter Rühren eine Lösung eines geeigneten Metallsalzes zugibt, wobei gegebenenfalls gekühlt wird, und die unter Dampf befindliche Kohle langsam, mindestens jedoch während einer Zeit von 1 h, auf Zimmertemperatur abkühlt und sie anschliessend trocknet.

8. Verfahren nach Anspruch 7, dadurch gekennzeichnet, dass man eine wässerige Lösung eines wasserlöslichen Metallsalzes, insbesondere eines Chlorids, Chromats oder Titanats, verwendet.

9. Hochspezifisches Adsorbens zur Durchführung des Verfahrens nach einem der Ansprüche 1 bis 3, dadurch gekennzeichnet, dass es 0 bis 50% hochporösen, pulverförmigen Feststoff, der mit 0,1 bis 5 Gew.-% Aluminium belegt ist, 0 bis 50 Gew.-% hochporösen, pulverförmigen Feststoff, der mit 0,1 bis 5 Gew.-% Zink belegt ist, und 0 bis 50 Gew.-% hochporösen Feststoff, der mit 0,1 bis 5 Gew.-% Chrom belegt ist, im Gemisch enthält, wobei der hochporöse Feststoff aus der Gruppe ausgewählt wird: Aktivkohle, Zeolithe, Metalloxide, insbesondere Aluminiumoxid oder Siliciumdioxid, mit einer spezifischen Oberfläche nach BET, bestimmt durch Argonadsorption bei −196°C, von 900 bis 1800 $m^2/g$, oder pyrogenem Siliciumdioxid mit einer spezifischen Oberfläche zwischen 50 und 500 $m^2/g$, bestimmt durch Argonadsorption bei −196°C, wobei sich die Gew.-%-Angaben jeweils auf das Gewicht des Feststoffs mit einer spezifischen Oberfläche von 1000 $m^2/g$ beziehen.

10. Hochspezifisches Adsorbens nach Anspruch 9, dadurch gekennzeichnet, dass es als hochporösen Feststoff Aktivkohle enthält.

11. Hochspezifisches Adsorbens nach Anspruch 9, dadurch gekennzeichnet, dass es 0 bis 50% Aktivkohle, die mit 0,15 bis 2,5 Gew.-% Aluminium belegt ist, 0 bis 50% Aktivkohle, die mit 0,25 bis 3,5 Gew.-% Zink belegt ist, und 0 bis 50% Aktivkohle, die mit 0,3 bis 5 Gew.-% Chrom belegt ist, im Gemisch enthält.

12. Hochspezifisches Adsorbens nach einem der beiden Ansprüche 10 oder 11, dadurch gekennzeichnet, dass es die mit Aluminium belegte Aktivkohle, die mit Zink belegte Aktivkohle und die

mit Chrom belegte Aktivkohle im Verhältnis 1:1:1 enthält.

## Revendications

1. Procédé d'adsorption et de décomposition catalytique de gaz intestinaux malodorants contenant de la vapeur d'eau, du méthanol, du monoxyde de carbone et du dioxyde de carbone et différents gaz soufrés, en particulier des mercaptans, caractérisé par le fait que l'on fait passer les gaz intestinaux à travers un solide inerte très poreux ayant une surface spécifique selon BET de 900 à 1800 $m^2/g$, déterminée par adsorption d'argon à −196°C, ou du dioxyde de silicium pyrogène ayant une surface spécifique selon BET de 50 à 500 $m^2/g$, déterminée par adsorption d'argon à −196°C, la surface des solides étant recouverte de 0,1 à 5% en poids d'aluminium, de zinc, de chrome ou de fer, relativement au poids du solide ayant une surface spécifique de 1000 $m^2/g$, et les composés soufrés ou composés du type mercaptan étant seuls décomposés catalytiquement et adsorbés et désorbés sous forme de fractions inodores.

2. Procédé selon la revendication 1, caractérisé par le fait que, comme solide, on utilise le charbon activé, des zéolites ou des oxydes métalliques, en particulier l'oxyde d'aluminium ou le dioxyde de silicium.

3. Procédé selon l'une des revendications 1 ou 2, caractérisé par le fait que l'on utilise du charbon activé ayant une surface spécifique selon BET de 1000 à 1800 $m^2/g$, dont la surface est couverte de 0,1 à 5% en poids d'aluminium, de zinc, de chrome ou de fer, relativement au poids du charbon activé ayant une surface spécifique de 1000 $m^2/g$, ou mélange de charbons activés de ce genre.

4. Procédé de fabrication d'un solide très poreux ayant une surface spécifique selon BET de 900 à 1800 $m^2/g$, déterminée par adsorption d'argon à −196°C, ou procédé de fabrication de dioxyde de silicium pyrogène ayant une surface spécifique selon BET de 50 à 500 $m^2/g$, déterminée par adsorption d'argon à −196°C, la surface des solides étant couverte de 0,1 à 5% en poids d'aluminium, de zinc, de chrome ou de fer, relativement au poids du solide ayant une surface spécifique de 1000 $m^2/g$, caractérisé par le fait qu'on lave le solide ou le dioxyde de silicium pyrogène avec de l'eau déminéralisée, qu'ensuite on le laisse à une température de 150 à 300°C sous un vide de $10^{-4}$ à $10^{-7}$ bar pendant un temps de 30 min à 10 h, qu'on ajoute alors au solide, sous vide, en agitant, une solution d'un sel approprié d'aluminium, de zinc, de chrome ou de fer, que l'on refroidit à la température ambiante le solide se trouvant sous de la vapeur, lentement mais au moins pendant un temps de 1 h et qu'ensuite on le sèche.

5. Procédé selon la revendication 4, caractérisé par le fait que l'on utilise comme solution une solution aqueuse d'un sel métallique hydrosoluble, en particulier d'un chlorure, chromate ou titanate.

6. Procédé selon l'une des revendications 4 ou 5, caractérisé par le fait qu'on lave le solide, pour le prétraitement, dans un bain d'ultrasons.

7. Procédé de fabrication d'un charbon activé très poreux ayant une surface spécifique selon BET de 1000 à 1800 $m^2/g$, déterminée par adsorption d'argon à −196°C, qui est couvert à sa surface de 0,1 à 5% en poids d'aluminium, de zinc, de chrome ou de fer, relativement au poids du charbon activé ayant une surface spécifique de 1000 $m^2/g$, caractérisé par le fait qu'on lave le charbon activé avec de l'eau déminéralisée, qu'ensuite on le laisse à une température de 150 à 300°C sous un vide de $10^{-4}$ à $10^{-7}$ bar pendant un temps de 30 min à 10 h et qu'alors, dans le vide, on ajoute au charbon, en agitant, une solution d'un sel métallique approprié, en refroidissant éventuellement et qu'on refroidit à la température ambiante le charbon placé sous de la vapeur, lentement mais pendant un temps d'au moins 1 h et qu'ensuite on le sèche.

8. Procédé selon la revendication 7, caractérisé par le fait que l'on utilise une solution aqueuse d'un sel métallique, en particulier d'un chlorure, chromate ou titanate.

9. Adsorbant très spécifique pour la mise en œuvre du procédé selon l'une des revendications 1 à 3, caractérisé par le fait qu'il contient, en mélange, 0 à 50% de solide pulvérulent très poreux qui est couvert de 0,1 à 5% en poids d'aluminium, 0 à 50% en poids de solide pulvérulent très poreux qui est couvert de 0,1 à 5% en poids de zinc et 0 à 50% en poids de solide très poreux qui est couvert de 0,1 à 5% en poids de chrome, le solide très poreux étant choisi dans le groupe comprenant: le charbon activé, les zéolites, les oxydes métalliques, en particulier l'oxyde d'aluminium ou le dioxyde de silicium, ayant une surface spécifique selon BET, déterminée par adsorption d'argon à −196°C, de 900 à 1800 $m^2/g$, ou le dioxyde de silicium pyrogène ayant une surface spécifique comprise entre 50 et 500 $m^2/g$, déterminée par adsorption d'argon à −196°C, les indications de pourcentage en poids étant chaque fois relatives au poids du solide ayant une surface spécifique de 1000 $m^2/g$.

10. Adsorbant très spécifique selon la revendication 9, caractérisé par le fait que comme solide très poreux il contient du charbon activé.

11. Adsorbant très spécifique selon la revendication 9, caractérisé par le fait qu'il contient 0 à 50% de charbon activé qui est couvert de 0,15 à 2,5% en poids d'aluminium, 0 à 50% de charbon activé qui est couvert de 0,25 à 3,5% en poids de zinc et 0 à 50% de charbon activé qui est couvert de 0,3 à 5% en poids de chrome.

12. Adsorbant très spécifique selon l'une des revendications 10 ou 11, caractérisé par le fait qu'il contient le charbon activé couvert d'aluminium, le charbon activé couvert de zinc et le charbon activé couvert de chrome en un rapport de 1:1:1.

## Claims

1. A process for the adsorption and catalytic decomposition of foul-smelling intestinal gases containing water vapor, methanol, carbon monoxide and carbon dioxide and various sulfur-containing gases, particularly mercaptans, characterized in that the intestinal gases are passed through an inert, highly porous solid having a specific BET surface of from 900 to 1800 $m^2/g$, as determined by argon adsorption at −196°C, or through pyrogenic silicon dioxide having a specific BET surface of from 50 to 500 $m^2/g$, as determined by argon adsorption at −196°C, the surface of the solids being covered with from 0.1 to 5% by weight of aluminium, zinc, chromium or iron, based on the weight of the solid having a specific surface of 1000 $m^2/g$, and only the sulfur and mercaptan compounds being catalytically decomposed and adsorbed and desorbed as non-smelling fractions.

2. A process as claimed in Claim 1, characterized in that active carbon zeolites or metal oxides, particularly aluminium oxide or silicon dioxide, are used as the solid.

3. A process as claimed in Claim 1 or 2, characterized in that active carbon having a specific BET surface of from 1000 to 1800 $m^2/g$, of which the surface is covered with from 0.1 to 5% by weight of aluminium, zinc, chromium or iron, based on the weight of the active carbon having a specific surface of 1000 $m^2/g$, or a mixture of active carbons such as these is used.

4. A process for the production of a highly porous solid having a specific BET surface of from 900 to 1800 $m^2/g$, as determined by argon adsorption at −196°C, or a process for the production of pyrogenic silicon dioxide having a specific BET surface of from 50 to 500 $m^2/g$, as determined by argon adsorption at −196°C, the surface of the solids being covered with from 0.1 to 5% by weight of aluminium, zinc, chromium or iron, based on the weight of the solid having a specific surface of 1000 $m^2/g$, characterized in that the solid or the pyrogenic silicon dioxide is washed with demineralized water and subsequently left standing in a vacuum of from $10^{-4}$ to $10^{-7}$ bar for 30 min to 10 h at a temperature of from 150 to 300°C, after which a solution of a suitable metal salt of aluminium, zinc, chromium or iron is added to the solid with stirring *in vacuo* and the solid now surrounded by vapor is cooled slowly, but at least over a period of 1 h, to room temperature and subsequently dried.

5. A process as claimed in Claim 4, characterized in that the solution used is an aqueous solution of a water-soluble metal salt, particularly a chloride, chromate or titanate.

6. A process as claimed in Claim 4 or 5, characterized in that the solid is pretreated by washing in a ultrasonic bath.

7. A process for producing a highly porous active carbon having a specific BET surface of from 1000 to 1800 $m^2/g$, as determined by argon adsorption at −196°C, which is covered over its surface with from 0.1 to 5% by weight of aluminium, zinc, chromium or iron, based on the weight of the active carbon having a specific surface of 1000 $m^2/g$, characterized in that the active carbon is washed with demineralized water and subsequently left standing in a vacuum of from $10^{-4}$ to $10^{-7}$ bar for 30 min to 10 h at a temperature of from 150 to 300°C, after which a solution of a suitable metal salt is added to the carbon with stirring *in vacuo*, optionally followed by cooling, and the carbon now surrounded by vapor is cooled slowly, but at least over a period of 1 h, to room temperature and subsequently dried.

8. A process as claimed in Claim 7, characterized in that an aqueous solution of a water-soluble metal salt, more particularly a chloride, chromate or titanate, is used.

9. A highly specific adsorbent for carrying the process claimed in any of Claims 1 to 3, characterized in that it contains in admixture from 0 to 50% of a highly porous, powder-form solid, which is covered with from 0.1 to 5% by weight of aluminium, from 0 to 50% by weight of a highly porous, powder-form solid, which is covered with from 0.1 to 5% by weight of zinc, and from 0 to 50% by weight of a highly porous solid which is covered with from 0.1 to 5% by weight of chromium, the highly porous solid being selected from the group comprising active carbon, zeolites, metal oxides, particularly aluminium oxide or silicon dioxide, having a specific BET surface, as determined by argon adsorption at −196°C, of from 900 to 1800 $m^2/g$, or pyrogenic silicon dioxide having a specific surface of from 50 to 500 $m^2/g$, as determined by argon adsorption at −196°C, all the percentages quoted being based on the weight of the solid having a specific surface of 1000 $m^2/g$.

10. A highly specific adsorbent as claimed in Claim 9, characterized in that it contains active carbon as the highly porous solid.

11. A highly specific adsorbent as claimed in Claim 9, characterized in that it contains in admixture from 0 to 50% of active carbon, which is covered with from 0.15 to 2.5% by weight of aluminium, from 0 to 50% of active carbon, which is covered with from 0.25 to 3.5% by weight of zinc, and from 0 to 50% of active carbon which is covered from 0.3 to 5% by weight of chromium.

12. A highly specific adsorbent as claimed in Claim 10 or 11, characterized in that it contains the aluminium-covered active carbon, the zinc-covered active carbon and the chromium-covered active carbon in a ratio of 1:1:1.

FIG.1

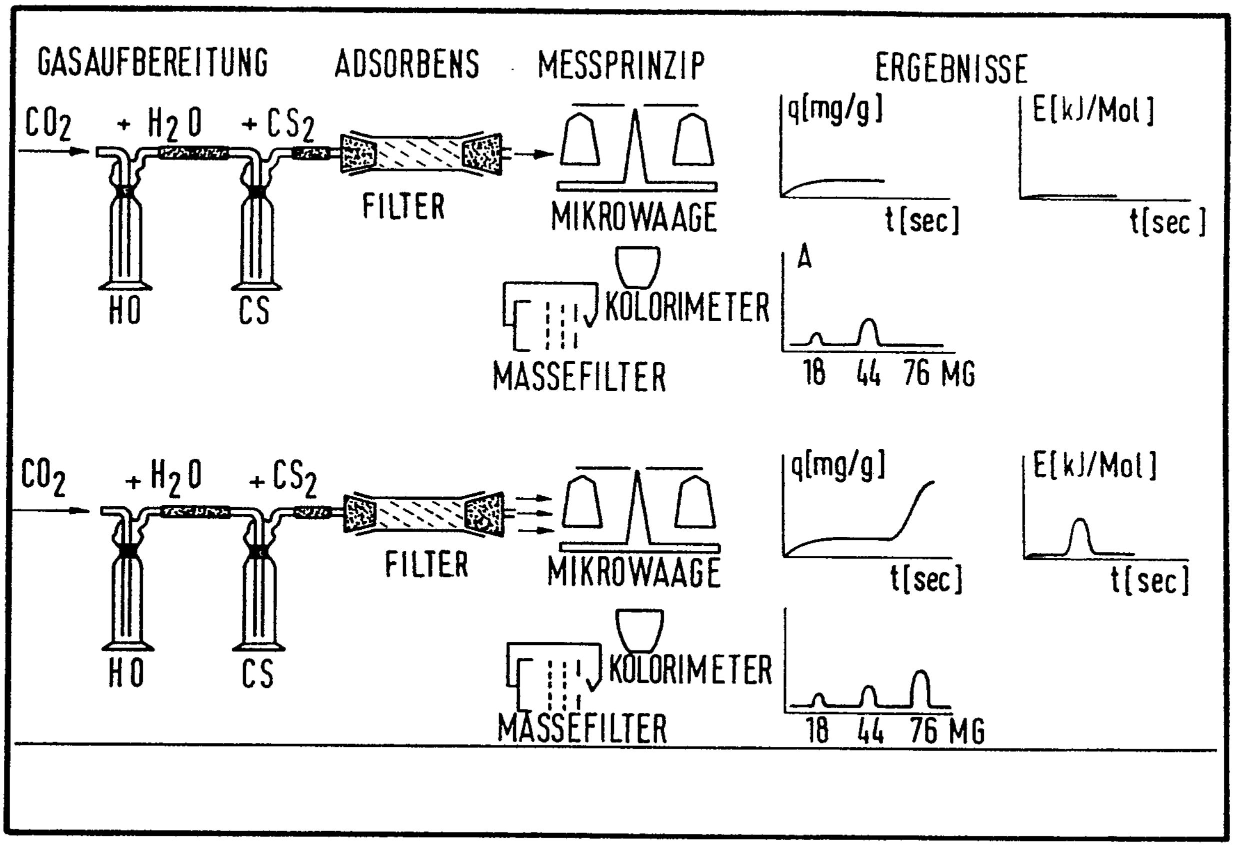


FIG.2

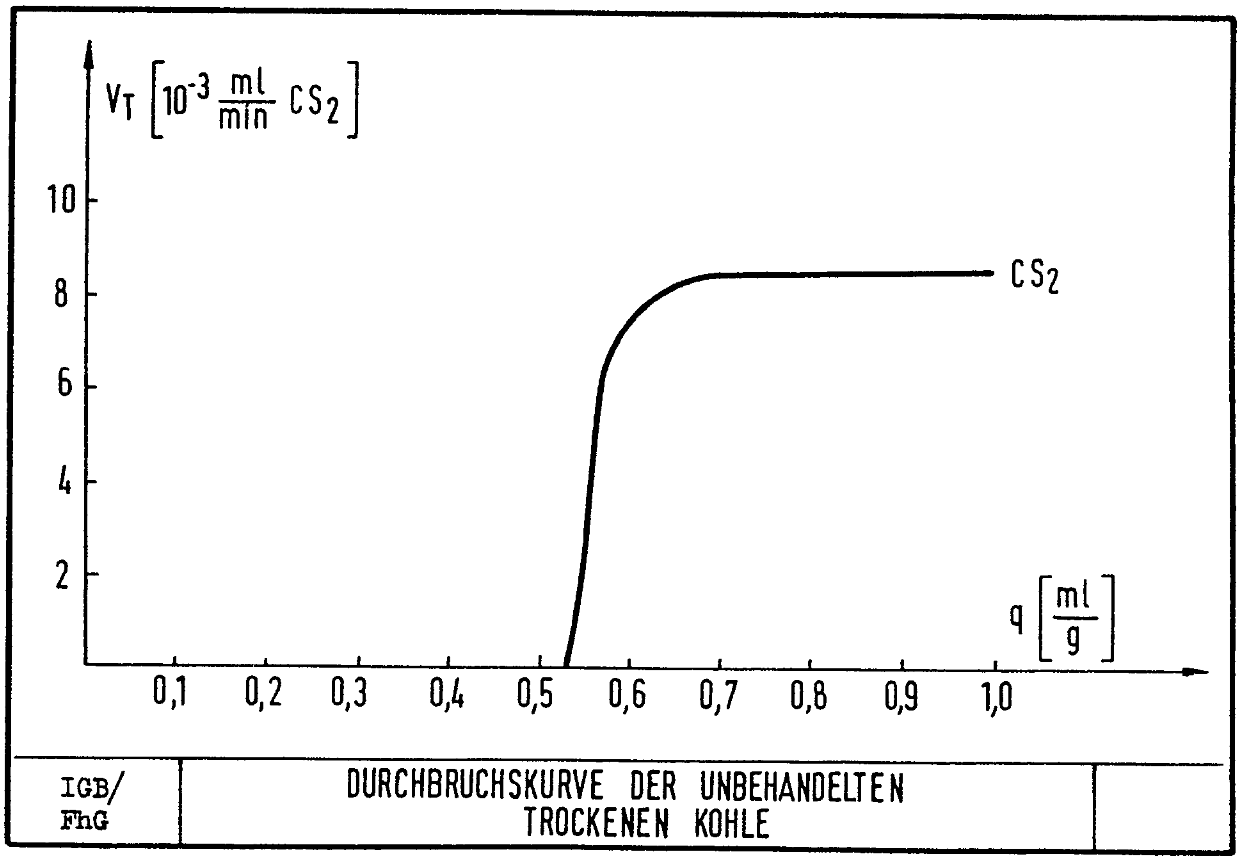


IGB/ FhG

DURCHBRUCHSKURVE DER UNBEHANDELTEN TROCKENEN KOHLE